(19) Europäisches Patentamt / European Patent Office / Office européen des brevets

(11) **EP 3 846 784 B1**

(12) **EUROPEAN PATENT SPECIFICATION**

(45) Date of publication and mention
of the grant of the patent:
**22.04.2026 Bulletin 2026/17**

(21) Application number: **19759623.2**

(22) Date of filing: **03.09.2019**

(51) International Patent Classification (IPC):
*A61K 9/20* (2006.01)        *A61K 9/28* (2006.01)
*A61K 47/38* (2006.01)        *A61P 1/00* (2006.01)
*A61K 31/606* (2006.01)

(52) Cooperative Patent Classification (CPC):
**A61K 9/286; A61K 9/205; A61K 31/606; A61P 1/00**

(86) International application number:
**PCT/EP2019/073462**

(87) International publication number:
**WO 2020/048979 (12.03.2020 Gazette 2020/11)**

(54) **CONTROLLED DRUG RELEASE FORMULATION**

GESTEUERTE WIRKSTOFFFREISETZUNGSFORMULIERUNG

FORMULATION À LIBÉRATION CONTRÔLÉE DE MÉDICAMENTS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.09.2018 EP 18192852
13.12.2018 EP 18212186**

(43) Date of publication of application:
**14.07.2021 Bulletin 2021/28**

(73) Proprietor: **Fachhochschule Nordwestschweiz
4132 Muttenz (CH)**

(72) Inventors:
• **IMANIDIS, Georgios
4103 Bottmingen (CH)**
• **LANZ, Michael
3084 Wabern (CH)**
• **LIPPS, Georg
4125 Riehen (CH)**
• **PAREDES, Valeria
4125 Riehen (CH)**

(74) Representative: **Bremi, Tobias Hans
Isler & Pedrazzini AG
Giesshübelstrasse 45
Postfach 1772
8027 Zürich (CH)**

(56) References cited:
EP-A1- 3 257 501     WO-A2-2009/047802

• MI KYONG YOO ET AL: "Drug release from xyloglucan beads coated with Eudragit for oral drug delivery", ARCHIVES OF PHARMACAL RESEARCH, 1 June 2005 (2005-06-01), Korea (South), pages 736 - 742, XP055558934, Retrieved from the Internet <URL:https://link. springer.com/content/pdf/10.1007/BF02969366. pdf> DOI: 10.1007/BF02969366
• ASHWINI R. MADGULKAR ET AL: "Optimization of Carboxymethyl-Xyloglucan-Based Tramadol Matrix Tablets Using Simplex Centroid Mixture Design", JOURNAL OF PHARMACEUTICS, vol. 2013, 1 January 2013 (2013-01-01), pages 1 - 11, XP055559137, ISSN: 2090-9918, DOI: 10.1155/ 2013/396468
• PANDIT A P ET AL: "Tamarind seed xyloglucan-based multilayer matrix tablet of tramadol hydrochloride for dual-release", DRUG DELIVERY LETTERS 20150701 BENTHAM SCIENCE PUBLISHERS B.V. NLD, vol. 5, no. 1, 1 July 2015 (2015-07-01), pages 63 - 71, XP009511411, ISSN: 2210-3031

**(Cont. next page)**

• MISHRA M U ET AL: "Evaluation of tamarind seed polysaccharide as abiodegradable carrier for colon specific drug delivery", vol. 3, no. 1, 1 January 2011 (2011-01-01), pages 139 - 142, XP009516752, ISSN: 0975-1491, Retrieved from the Internet <URL:https://innovareacademics.in/journal/ijpps/Vol3Issue1/1012.pdf>
• A.M.J. NEWTON ET AL: "Chronotherapeutic drug delivery of Tamarind gum, Chitosan and Okra gum controlled release colon targeted directly compressed Propranolol HCI matrix tablets and in-vitro evaluation", INTERNATIONAL JOURNAL OF BIOLOGICAL MACROMOLECULES., vol. 79, 1 August 2015 (2015-08-01), NL, pages 290 - 299, XP055635037, ISSN: 0141-8130, DOI: 10.1016/j.ijbiomac.2015.03.031

**Description**

TECHNICAL FIELD

The present invention relates to pharmaceutical formulation dosage forms suitable for (per)oral administration, in particular suitable for the oral administration of active pharmaceutical ingredients to be delivered selectively to the colon as well as methods for making such pharmaceutical formulation dosage forms and dosage regimens suitable for the corresponding dosage forms.

PRIOR ART

WO-A-2015158771 discloses compositions comprising synergic combinations of xyloglucans and plant or animal proteins, which are useful in the treatment of intestinal disorders. Tablets for the treatment of diarrhea are proposed based on xyloglucan, pea protein or gelatin.

US-A-2017088557 describes a process for the preparation of rifaximin $\tau$, an antibiotic used to treat traveler's diarrhea, irritable bowel syndrome, and hepatic encephalopathy, a pharmaceutical composition comprising said rifaximin form as well as typical formulation ingredients such as microcrystalline cellulose, HPMC, glyceryl stearate, sodium starch glycolate, and its use for treating inflammations and infections.

WO-A-2007122374 discloses a delayed release coating comprising a mixture of a first material selected from starch; amylose; amylopectin; chitosan; chondroitin sulfate; cyclodextrin; dextran; pullulan; carrageenan; scleroglucan; chitin; curdulan and levan, and a second material which has a pH threshold at about pH 5 or above, is used to target release of a drug from a core to the intestine, particularly the colon.

US-A-2018000740 discloses pharmaceutical particulates which release a pharmaceutical compound into the colon following oral administration. A particulate comprises a core comprising a pharmaceutical compound, an inner coating surrounding the core, wherein the inner coating comprises a pharmaceutically acceptable polysaccharide that is susceptible to enzymatic digestion by one or more enzymes present colonic microflora, and an outer coating surrounding the inner coating, wherein the outer coating comprises a polymer which is stable at upper gastrointestinal pH but can dissolve at pH>6. The core of a particulate can further comprise an excipient such as a diluent, a binder, a disintegrant, a lubricant, a glidant or a combination thereof. Particulates can comprise pharmaceutical compounds for treating colonic diseases such as C. difficile infection, ulcerative colitis, colon cancer, and Crohn's disease.

Paulraj et al in "Bioinspired capsules based on nano-cellulose, xyloglucan and pectin - The influence of capsule wall composition on permeability properties" (Acta Biomaterialia 69 (2018) 196-205) present a study in which hollow microcapsules are built up in a layer by layer process using cellulose nano-fibrils and xyloglucan-amyloid or cellulose nano-fibrils, xyloglucan-amyloid and apple pectin as material for the individual layers. It is found that the corresponding wall structure is selectively permeable, depending on the electrolyte concentration, to a system such as dextrane and use of the corresponding microcapsules for pharmaceutical purposes are envisaged as future applications.

Mishra et al. (Int J Pharm Sci, 3(1), 139-142) describe the use of tamarind seed polysaccharide as tablet matrix and studied the release of ibuprofen in in-vitro test. Ibuprofen release is accelerated in the presence of rat caecal content.

Svagan et al in "Rhamnogalacturonan-I Based Microcapsules for Targeted Drug Release" (PLOS ONE, December 19, 2016) disclose a method for making microcapsules based on Rhamnogalacturonan-I cross-linked by a diisocyanate and provide evidence that the corresponding microcapsules can uptake model systems as well as evidence that the microcapsules are released under corresponding enzymatic conditions.

Yoo et al. (Arch Pharm Res Vol 28, 6, p736 -742, 2005) describes the use of a degalactosylated xyloglucan for the sustained release of indomethacin. By treatment of xyloglucan with a beta-galactosidase the terminal galactose residues are removed leading to a change of the rheological and colloidal properties of the polymer. Degalactosylated xyloglucan exhibits thermally reversible sol-gel transitions a property not observed with unmodified xyloglucan (Brun-Graeppi, Amanda K. Andriola Silva et al. 2010. "Study on the Sol-Gel Transition of Xyloglucan Hydrogels." Carbohydrate Polymers 80(2): 555-62.). The degalactosylated xyloglucan was mixed with indomethacin and allowed to form hydrogels. The hydrogel beads were dried and coated with Eudragit L100. The obtained formulation mainly released indomethacin in the small intestine as shown by in-vitro experiments simulating the gastric passage.

WO-A-2012038898 discloses gastro-resistant tablets containing rifaximin, obtained by means of gastro-resistant micro-granules characterized in that they inhibit the rifaximin release at pH values between 1.5 and 4.0, and they allow its release at pH values between 5.0 and 7.5, the processes for their obtainment and their use in the treatment and the prevention of diseases directly or indirectly deriving from inflammatory bowel diseases. The active pharmaceutical ingredient is embedded in a matrix of various constituents, including silica, methacrylic acid methyl methacrylate, talc, titanium dioxide, iron oxide, microcrystalline cellulose, magnesium stearate, et cetera. The tablets may be provided with a film coating based on hydroxy propyl methylcellulose and titanium dioxide.

Magdulkar et al. (Journal of Pharmaceutics, vol. 2013, 1 January 2013, pages 1-11) disclose the optimization of Carboxymethyl-Xyloglucan-Based Tramadol Matrix Tablets Using Simplex Centroid Mixture Design.

Pandit et al. (Drug Delivery Letters, 20150701, Bentham Science Publishers B.V. NLD, vol. 5, no. 1, 1 July 2015, pages 63-71) report Tamarind seed xyloglucan-based multilayer matrix tablets of tramadol hydrochloride for dual-release. WO-A-2009/047802 relates to a colon targeted modified release bioadhesive pharmaceutical composition of 5-amino salicylic acid or a pharmaceutically acceptable salt or enantiomer or polymorph or metabolites thereof.

EP-A-3 257 501 relates to a multiple unit dosage form comprising a core, wherein the core comprises a plurality of individual units, each unit comprising a pharmaceutically active ingredient and each unit being covered with a mucoadhesive material, and wherein the multiple unit dosage form further comprises an enteric core coating layer.

Mishra et al (International Journal of Pharmacy and Pharmaceutical Sciences, Madhya Pradesh, in vol. 3, no. 1, 1 January 2011, pages 139-142) relates to the evaluation of tamarind seed polysaccharide as a biodegradable carrier for colon specific drug delivery.

Newton et al. (International Journal of Biological Macromolecules, vol. 79, 1 August 2015, pages 290-299) discloses chronotherapeutic drug delivery of Tamarind gum, Chitosan and Okra gum controlled release colon targeted directly compressed Propranolol HCI matrix tablets and in-vitro evaluation.

SUMMARY OF THE INVENTION

It is therefore an object of the present invention to provide and propose a new pharmaceutical formulation dosage form suitable for use for (per)oral administration and allowing for a targeted release of an active pharmaceutical ingredient (API), in particular in the colon, or suitable for immunomodulation or immunosuppression or suitable for establishing, re-establishing and/or modifying the balance of the microbiome population in the colon or the physiology of the lower gastrointestinal tract.

The proposed pharmaceutical formulation dosage form as claimed in claim 1 is a core-shell type tablet which comprises a core encapsulated by at least one shell and at least one active pharmaceutical ingredient, wherein the at least one active pharmaceutical ingredient is embedded in said core of the pharmaceutical formulation dosage form.

According to the present invention, at least one of said core and said shell is, at least partly, based on native, highly purified cold-water soluble type xyloglucan. Preferably said core is formed by a matrix based on said native, highly purified cold-water soluble type xyloglucan, or essentially consisting of said native, highly purified cold-water soluble type xyloglucan, containing said active pharmaceutical ingredient. Said native, highly purified cold-water soluble type xyloglucan is thus acting as an excipient and/or additive in solid pharmaceutical dosage forms for oral / per-oral administration, including tablets, e.g. compressed tablets or molded tablets, which can be un-coated, film-coated or sugar coated, to control and target delivery of active pharmaceutical ingredients for local therapeutic action in the gastrointestinal tract including the colon.

Furthermore, said shell is a pH-responsive coating, and preferably said native, highly purified cold-water soluble type xyloglucan, if only in the shell, should be in the layer which is forming the pH-responsive coating or should be in a coating layer which is inside of the pH-responsive coating.

The invention thus entails the use of native, highly purified cold-water soluble type xyloglucan as a matrix forming material for the manufacture of solid dosage forms such as tablets in which active pharmaceutical ingredient (API) is physically embedded. When in contact with intestinal fluid, the native, highly purified cold-water soluble type xyloglucan matrix or coating does not disintegrate instead slowly forming a highly viscous gel-like solution or gluey mass that impedes the release of API. Upon arrival of the dosage form in the colon, said native, highly purified cold-water soluble type xyloglucan is digested by the microflora triggering release of the API. Hence, API or drug delivery specifically to the colon is achieved eliciting efficient API targeting.

Xyloglucan is a polysaccharide of plant cell wall origin. A xyloglucan quality purified from Tamarindus indica seeds is used although material of other plant sources may also apply. Xyloglucan was shown to be digested by several Bacteroides species which is the most abundant genus in the gut microbiome.

To minimize contact of said native, highly purified cold-water soluble type xyloglucan with intestinal fluid before it reaches the colon and further lessen premature release of API, the dosage form is coated with a pH-responsive film that dissolves at pH of at least 6.8.

The combination of said native, highly purified cold-water soluble type xyloglucan used as embedding matrix material for API with pH sensitive film coating creates a redundancy of release controlling mechanisms that is intended to optimize the therapeutic index of the API. The coating is designed to dissolve at a slightly acidic to neutral pH that occurs under all circumstances in the small intestine to absolutely assure that the film is removed before the dosage form reaches the colon. After the coating is dissolved, release of API that would otherwise take place prematurely in the small intestine is impeded by the property of said native, highly purified cold-water soluble type xyloglucan to not disintegrate forming instead a highly viscous mass. Only digestion of said native, highly purified cold-water soluble type xyloglucan by the colonic microbiota triggers release of the API providing highly efficient active ingredient or drug targeting.

Controlled release in the gastrointestinal tract relying only on pH-sensitive coatings provides highly variable results. This is due to the intra and inter-individual variability of pH in the intestine, the dependence of pH on the intake of food, etc. Thus, an early dissolution of the coating before the arrival of the dosage form in the large intestine results in systemic absorption of the active ingredient and therefore its loss for colon specific delivery and local therapeutic effect, and the generation of systemic side effects, i.e., a worsened therapeutic index. A failure of the coating to dissolve in the small intestine on the other hand results in elimination of the intact dosage form in the faeces.

Use of materials forming a matrix as a means to prevent release of active ingredient or drug substance in the small intestine requires effective barrier formation by these materials in the aqueous environment of the small intestine and their efficient degradation by the microbiome of the colon.

Prior art has used enteric coating alone and xyloglucan alone to prevent release in the stomach and to enhance release in the intestine but failed to demonstrate the combination of the dual use of enteric coating and xyloglucan is useful for colonic delivery. The proposed dual release mechanism also specifically addresses the intra- and inter-individual variability of the gut conditions for which no solutions have yet been proposed.

Efficient active ingredient or drug targeting to the colon by per-oral administration with minimal active ingredient or drug release in the small intestine and therefore minimal absorption into the systemic circulation and maximal delivery of API in the colon for local therapeutic effect is thus achieved. This is required and advantageous for therapeutic treatment of inflammatory bowel disease, colon cancer, clostridium difficile infection and further conditions of the large intestine that benefit from local rather than systemic active ingredient or drug application, but also for immunomodulation or immunosuppression or for the purpose of establishing, re-establishing and/or modifying the balance of the microbiome population in the colon or the physiology of the lower gastrointestinal tract.

New pharmaceutical products are thus made available for specific colonic active ingredient or drug delivery by per-oral administration. Therapeutic areas include inflammatory bowel disease and colon cancer, but also immunomodulation or immunosuppression. Existing active pharmaceutical ingredients (API) for these indications may primarily be used, although utilization of new chemical entities is also possible.

The expression active pharmaceutical ingredient (API) in the context of the present application includes conventional pharmaceutical compounds, be it small molecules or large molecules such as for example antibody-based pharmaceuticals, in particular for treatment as a tablet or for immunomodulation or immunosuppression.

However active pharmaceutical ingredient in the present context also includes any kind of material for the purpose of establishing, re-establishing and/or modifying the balance of the microbiome population in the colon. These include:

1) selected strains of bacteria including spores and/or mixture of strains;
2) nutrients for colonic microorganisms like fermentation substrates, nitrogen source, trace elements (Fe) etc.;
3) modulators of bacterial growth including vitamins, hormones, antibiotics, toxins etc.;
4) compounds which influence the composition of the microbiome by favoring and/or disfavoring the growth, viability or colonization of selected strains.

So the term API also generally includes compounds which have a beneficial effect on the physiology of the lower gastrointestinal tract.

Current delivery modalities do not achieve the level of colon targeting that is required for best active ingredient or drug therapeutic index comprising maximal therapeutic effect or immunomodulation or immunosuppression or re-establishing and/or modifying the balance of the microbiome population, and minimized side effects.

Depending upon the porosity of the tablet, as can be seen further below, after a short burst the API (5-ASA) is released with zero order kinetics afterwards. The same release kinetics is observed regardless whether the tablets are coated or not. Uncoated tablets release 5-ASA immediately; coated tablets release as soon as the enteric coatings is dissolved (depending upon pH and specific coating type and thickness used).

Tablets with enteric coating use the coating to protect the tablet and to prevent disintegration and API release during the gastric passage. Commercial 5-ASA tablets mostly have a poly-(acrylate-methacrylate) coating. The coatings differ in composition and the release is specifically triggered at a certain time point depending on the pH which initiates the dissolution of the coating. Thus, the time point of release is critically dependent upon pH. In other words, the delay intended by the tablets is largely influenced by the pH in the intestine, which in turn is influenced by a number of physiological factors. Consequently, a reliable delayed release with such a formulation is not attainable especially in patients suffering Morbus Crohn and IBD.

In contrast, the tablets proposed here are formulated in such a way to prevent release under weakly acidic to almost neutral conditions as long as possible. This in principle bears the risk that the tablets do not disintegrate in the colon and are excreted more or less intact. However, the inclusion of said native, highly purified cold-water soluble type xyloglucan as tablet matrix destabilizes the tablet core in the colon due to the action of the microbiome which specifically digests plant cell wall material. The purpose of the enteric coating for our technology is therefore to allow a stabilization of the tablet also along the small intestine. During the gastric and the small intestine passage the matrix core is wetted which could speed up the digestion of the native, highly purified cold-water soluble type xyloglucan in the colon by the resident colonic microbiome.

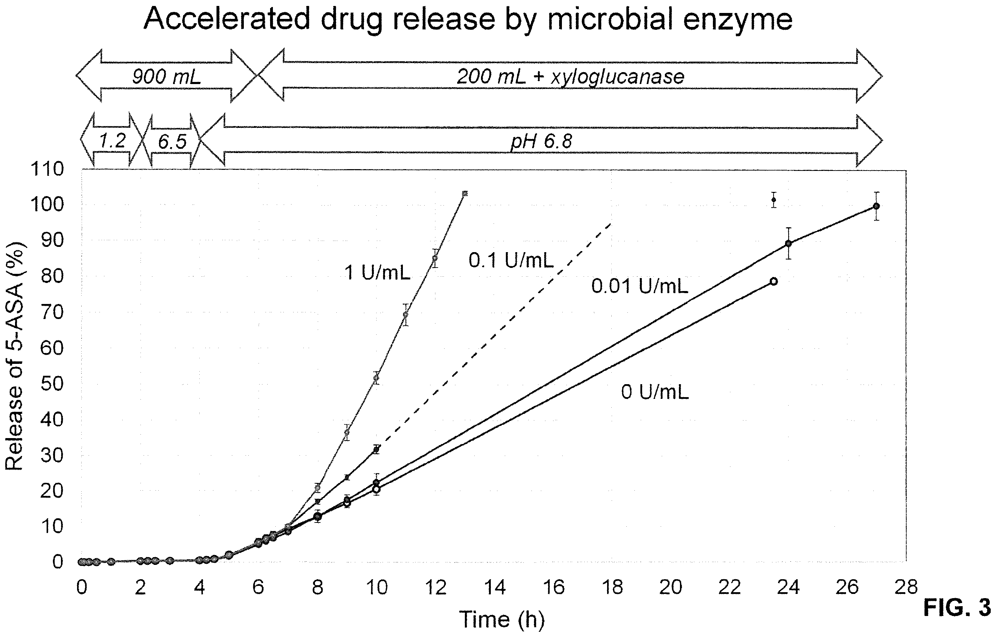

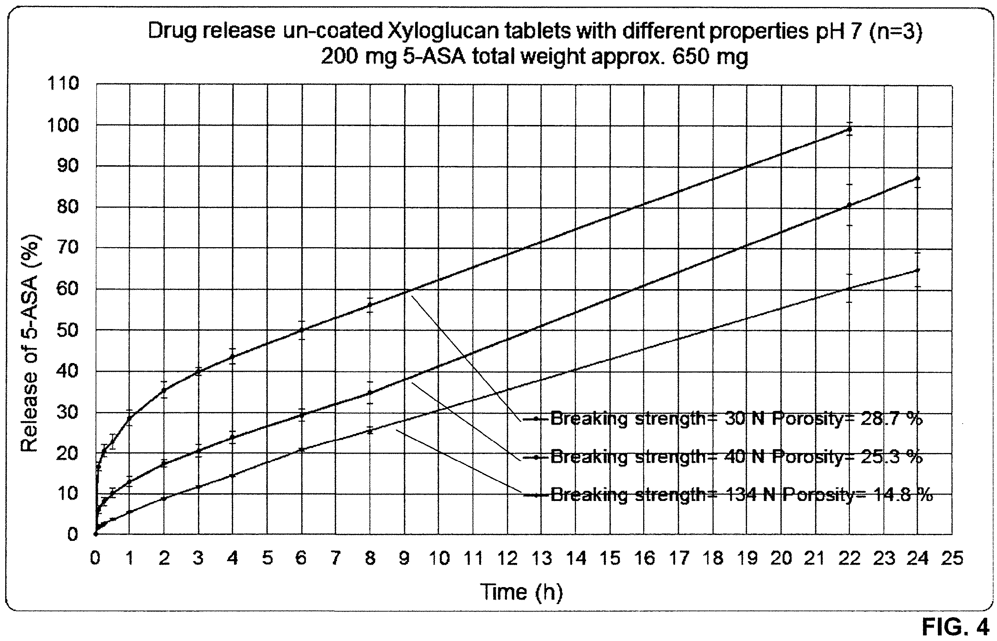

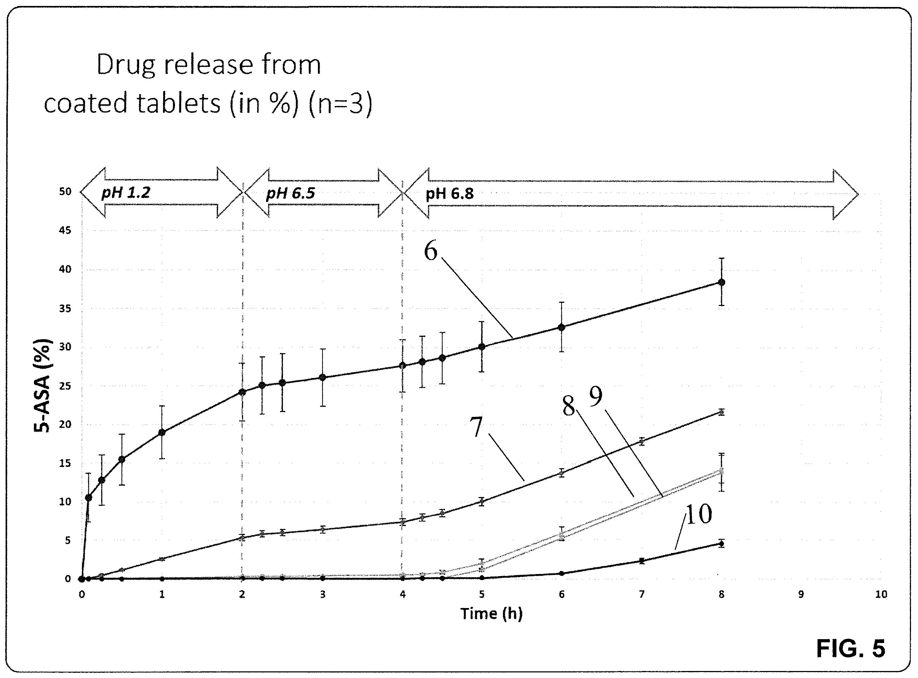

The combination of enteric coating and said native, highly purified cold-water soluble type xyloglucan goes beyond a simple additive effect. Surprisingly, there is a synergetic effect on API (e.g. 5-ASA) release. The coated and the uncoated tablets release 3 to 4% of the API (e.g. 5-ASA) load per hour in the absence of the microbial enzyme (Fig. 4 (uncoated), Fig. 3, trace 0 U/mL and Fig 5, trace 8 and 9 (coated)). In contrast, the uncoated tablets release under the same experimental conditions but in the presence of the microbial enzyme 7.5% of API (5-ASA) per hour and the coated tablet 15 % per hour (Fig. 3, trace 1 U/mL). This more rapid release observed in the coated tablets allows for a more efficient colonic delivery. The pharmaceutical formulation dosage form, typically a tablet, is preferably adapted for oral administration and for targeted release of the active pharmaceutical ingredient in the colon. To this end, preferably said shell is a pH-responsive coating dissolving only at a pH of more than 6.5, preferably of at least 6.7, more preferably of at least 6.8.

According to a first preferred embodiment, said at least one active pharmaceutical ingredient is embedded in said core of the pharmaceutical formulation dosage form in that said core is formed by a matrix based on said native, highly purified cold-water soluble type xyloglucan containing said active pharmaceutical ingredient. So the shell may be free from xyloglucan, the API then being embedded in the core in a matrix based on or essentially consisting of said native, highly purified cold-water soluble type xyloglucan.

Said shell may comprise alternatively or additionally at least one outer layer in the form of a pH responsive coating with at least one layer based on said native, highly purified cold-water soluble type xyloglucan. If the shell comprises a layer based on said native, highly purified cold-water soluble type xyloglucan, typically this is instead of having said native, highly purified cold-water soluble type xyloglucan as the matrix component of the core. The core is then preferably formed by the API alone or the core contains the API in a matrix without xyloglucan. However it is also possible to have a shell layer based on said native, highly purified cold-water soluble type xyloglucan as well as a core matrix based on said native, highly purified cold-water soluble type xyloglucan.

In case of a shell layer based on said native, highly purified cold-water soluble type xyloglucan said shell layer based on said native, highly purified cold-water soluble type xyloglucan or further shell layers then include further components to provide for the pH-responsivity. In particular for the case where a pH-responsive outer coating of the shell is not based on xyloglucan, e.g. for the case where there is no xyloglucan forming the matrix of the API in the core, there can be at least one further inner shell layer based on said native, highly purified cold-water soluble type xyloglucan.

The pharmaceutical formulation dosage form can be adapted for oral administration and for targeted release of the active pharmaceutical ingredient in the colon, and said shell may comprise at least one or consist of a pH-responsive coating dissolving only at a pH of more than 6.5, preferably of at least 6.7, more preferably of at least 6.8.

Said shell, in particular the at least one pH responsive coating thereof, can be based on synthetic polymers such as an anionic acrylate copolymer, preferably on an anionic copolymer based on methyl acrylate, methyl methacrylate and methacrylic acid, wherein preferably the ratio of the free carboxyl groups to the ester groups is in the range of 1:5 - 1:10, preferably in the range of 1:10, wherein preferably the anionic acrylate copolymer has a weight average molar mass (Mw) in the range of 200'000-400'000 g/mole, preferably in the range of 250'000-300'000 g/mole, one or a mixture of the following systems: biopolymers, in particular non watersoluble biopolymers, such as plant and/or animal derived biopolymers, including mixtures of free and esterified aliphatic and/or aromatic hydroxyacids,

Said shell, in particular the at least one pH responsive coating thereof, may consist of a mixture of an anionic acrylate copolymer, preferably on an anionic copolymer based on methyl acrylate, methyl methacrylate and methacrylic acid, wherein preferably the ratio of the free carboxyl groups to the ester groups is in the range of 1:5 - 1:10, preferably in the range of 1:10, wherein preferably the anionic acrylate copolymer has a weight average molar mass (Mw) in the range of 200'000-400'000 g/mole, preferably in the range of 250'000-300'000 g/mole, with further additives in a proportion of less than 25%, said further additives preferably being selected from the group consisting of polyoxyethylene and derivatives thereof, anionic surfactants, in particular sodium laurylsulfate, talc, dye, in particular iron(III)oxide, stabilizers, in particular triethyl citrate,

The dry coating amount of the at least one pH responsive coating or of the whole shell can be in the range of 1-10, preferably in the range of 2.5-6 mg/cm2. In particular if the shell or at least one layer of the shell is based on said native, highly purified cold-water soluble type xyloglucan the dry coating amount can also be much higher. For example, if the core is made of API alone, then the native, highly purified cold-water soluble type xyloglucan shell layer can be, by weight, up to as much as the core, e.g. in the range of 30-50% by weight of the core.

There can be provided only one single encapsulating pH responsive coating forming said shell.

Said matrix of the core may essentially or completely consist of said native, highly purified cold-water soluble type xyloglucan, wherein preferably said native, highly purified cold-water soluble type xyloglucan is obtained from Tamarindus indica seeds and/or is amorphous.

The native, highly purified cold-water soluble type xyloglucan used as starting material can have a particle size (d50%) of at least 70 $\mu$m, preferably in the range of 70-150 $\mu$m, more preferably in the range of 80-110 $\mu$m.

The native, highly purified cold-water soluble type xyloglucan can have a weight average molar mass (Mw) in the range of 400,000-500,000 g/mol.

Said native, highly purified cold-water soluble type xyloglucan is fully cold water soluble, meaning it is fully soluble upon cold mixing of the starting materials at room temperature for a concentration of at least 1% w/v, preferably of at least 1.5 or 2% w/v in distilled water.

**Preferably** this type of said xyloglucan is further fully amorphous and essentially free from impurities, in particular free from glucose and/or dextran, i.e. the purity of the starting material is at least 90% by weight, preferably a least 95% or at least 99%. If such a type of said native, highly purified cold-water soluble type xyloglucan is chosen the tablets do have a reduced tendency of disintegration and are thus more stable and provide for a more consistently controlled and reliable API release in the colon. In particular, tablets can be made which do not disintegrate even after 4h or 6h in water at room temperature (measured according to Ph.Eur.).

Surprisingly, the type of xyloglucan has a significant effect on its effect and suitability. We have worked with two highly purified but otherwise native xyloglucans Glyloid 2A (hot water soluble) and 3S (cold water soluble). The hot water soluble variety Glyloid 2A was processed for tablet core production. The tablet cores disintegrated rapidly. We have expected that the hot water soluble xyloglucan would be more suitable than the cold water soluble xyloglucan since the hot water soluble was expected to release the API less efficiently at room or body temperature. So one would expect that the hot-water soluble variety would be the preferred matrix as it would not dissolve at physiological pH and form a solid matrix impeding drug release while the cold-water-soluble variety would entail the considerable risk that the matrix would rapidly dissolve away in the intestine rendering a delayed or colonic delivery impossible. Surprisingly we found that tablets produced from hot-water soluble types disintegrated in water relatively fast (< 1 hour) into small solid particles which due to the high surface then release the API rather quickly, while in contrast, the cold-water soluble xyloglucan tablets formed a viscous gluey mass in the perimeter that impeded drug release while the tablet remained intact for at least 24 hours.

Preferably the xyloglucan is therefore non-degalactosylated.

According to the invention, the xyloglucan is native, highly purified cold-water soluble type xyloglucan.

The core may consist of

(A) 25 - 90%, preferably 40 - 90% by weight of said native, highly purified cold-water soluble type xyloglucan;
(B) 10 - 60% by weight of at least one active pharmaceutical ingredient;
(C) 0 - 20% by weight, preferably 5 - 10% by weight of one or more pharmaceutically acceptable excipients selected from the group consisting of a diluent, a binder, an anti-adherent, a lubricant, a glidant and a combination thereof, wherein preferably the pharmaceutically acceptable excipient essentially consists of a binder or a binder and an anti-adherent, in particular the binder being selected as PVP and the anti-adherent as magnesium stearate.

The core may also consist of granules consisting of

(A) 25 - 90%, preferably 40-90% by weight of said native, highly purified cold-water soluble type xyloglucan;
(B) 10-60% by weight of at least one active pharmaceutical ingredient;
(C) 0-20% by weight, preferably 5-10% by weight of one or more pharmaceutically acceptable excipients selected from the group consisting of a diluent, a binder, a lubricant, a glidant and a combination thereof, wherein preferably the pharmaceutically acceptable excipient essentially consists of a binder, in particular the binder being selected as PVP,

which granules are compacted to form a core (before applying the shell, wherein preferably before compacting the granules are blended with an anti-adherent, preferably in the form of magnesium stearate.

Preferably the core is a single solid compressed core with a relative density of at least 0.7 (70%), preferably of at least 0.75 (75%) or at least 0.8 (80%).

The apparent density is determined based on the weight of the tablet (by weighing, room temperature, 23°, r.H. 65%) and the volume of the tablet calculated from the geometric form by geometric formulas.

The relative density ($\rho_r$) or porosity is calculated by the following formula

$$\rho_r = 1 - \varepsilon = \frac{\rho_{sch}}{\rho_{solid}}$$

wherein the apparent density ($\rho_{sch}$) is determined from the weight and the volume (as above) and the solid density ($\rho_{solid}$) is measured by a gas pycnometer (model used here is a Multi-Pycnometer available from Quantachrome Instruments). The method with the gas pycnometer is e.g. described in the European Pharmacopoeia Ph.Eur. 8 (2.9.23, p 324).

Preferably the core and/or the whole pharmaceutical formulation dosage form has an average extension in the direction of the smallest diameter of at least 3 mm, preferably at least 4 mm, more preferably at least 4.5 or 5 mm. Preferably they have an average extension in the direction of the largest diameter of at least 8 mm, preferably of at least 10mm, more preferably in the range of 10-14mm or of 12 mm. The tablets are preferably compressed or moulded tablets and they can be of circular, oval or polygonal, in particular rectangular with rounded edges shape in the direction of the larger extension, and they can be flat faced plain, flat faced radius edged, flat faced bevel edged, standard convex face, compound cut. Inter alia for the mere size there is delay of release: Release from a spherical matrix with a radius of 1 and an active ingredient content of 570 (arbitrary units) shows a total release reached after 140 hours. A matrix half the size with radius 0.5 has an active ingredient content of 70 (due to the smaller volume, with identical density and other parameters) and releases the total amount of active ingredient in 40 hours. A matrix with a radius of 0.2 has an active ingredient content of 4.5 and shows a complete release in 6 hours. These are simulated (calculated) results based on the diffusion equation and demonstrate that normalized to the same total quantity of active ingredient, the larger (geometrically) the dosage form (tablets, beads, etc.), the slower the release process.

Preferably the core and/or the whole pharmaceutical formulation dosage form has a crushing force of at least 25 N, preferably of at least 40 N, more preferably of at least 100 N. The method for determining the crushing force of the tablets is also described in the European Pharmacopoeia Ph.Eur. 8 (2.9.8. p 299).

The weight ratio of the matrix of the core matrix based on said native, highly purified cold-water soluble type xyloglucan to the at least one active pharmaceutical ingredient is preferably at least 1:2, preferably at least 1:1, more preferably at least 2:1.

The porosity of the core with said native, highly purified cold-water soluble type xyloglucan as matrix can be in the range 10 - 35% (void volume percentage), and the degree of porosity can be used to control the release properties of the API.

The proposed pharmaceutical formulation dosage form can be suitable for establishing, re-establishing and/or modifying the balance of the microbiome population in the colon or the physiology of the lower gastrointestinal tract, or it can be for use for immunomodulation or immunosuppression, or for the treatment of at least one of the following conditions: inflammatory bowel disease, in particular ulcerative colitis and/or Crohn's disease, Clostridium difficile infection, colon cancer, post colon surgical treatment.

The active pharmaceutical ingredient can be selected from the group consisting of: mesalazine, budesonide, capecitabine, fluorouracil, irinotecan, oxaliplatin, UFT, cetuximab, panitumumab. UFT is a dihydropyrimidine dehydrogenase inhibitory fluoropyrimidine drug, which combines uracil, a competitive inhibitor of DPD, with the 5-fluorouracil (5-FU) prodrug tegafur in a 4:1 molar ratio.

Further possible are immunomodulatory or immunosuppressive ingredients including immunosuppressive glucocorticoids, immunosuppressive cytostatics, immunosuppressive (poly- or monoclonal) antibodies, immunosuppressive drugs acting on immunophilins, interleukins, cytokines, chemokines, immunomodulatory imide drug. Possible are e.g. in particular tacrolimus, cyclosporine.

Also possible active pharmaceutical ingredients are materials for the purpose of establishing, re-establishing and/or modifying the balance of the microbiome population in the colon or compounds which have a beneficial effect on the physiology of the lower gastrointestinal tract, or combinations thereof.

The pharmaceutical formulation dosage form suitable for oral administration can be administered orally at least once a day, preferably twice a day, over a time span of at least one week, preferably at least two weeks, or at least two months or at least 1 year or even life-long.

Furthermore the present invention relates to a method for making a pharmaceutical formulation dosage form as given above, wherein in a first step said native, highly purified cold-water soluble type xyloglucan, at least one active pharmaceutical ingredient, as well as if needed one or more pharmaceutically acceptable excipients are mixed and then compacted to form the core or mixed and treated to form granules, which are subsequently, if needed by first mixing the granules with a further treatment agent, compacted to form the core, wherein the mixing in both cases can take place using preferably a fluidised bed granulator or high shear mixer, and the core is subsequently coated in a second step with at least one coating forming a shell, wherein preferably the coating formulation is provided as a dispersion and is applied further preferably in a drum coater or using another method.

Further embodiments of the invention are laid down in the dependent claims.

BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,

Fig. 1     shows a schematic representation of the action mechanism;
Fig. 2     shows the release profile of different concentrations of the API in the matrix over time and conditions with mesalazine as API (5-ASA);
Fig. 3     shows the release profile of API in the presence of different concentrations of the xyloglucanase in the solution in the last phase over time and conditions with mesalazine as API (5-ASA);

Fig. 4    shows the property of xyloglucan to slow down active ingredient or drug release depending on the porosity of un-coated tablets;

Fig. 5    shows the release profile of API in the presence of different types of tablets with different thickness (amount) of the coating in solutions simulating the conditions of passage through the gastrointestinal tract over time with mesalazine as API (5-ASA).

DESCRIPTION OF PREFERRED EMBODIMENTS

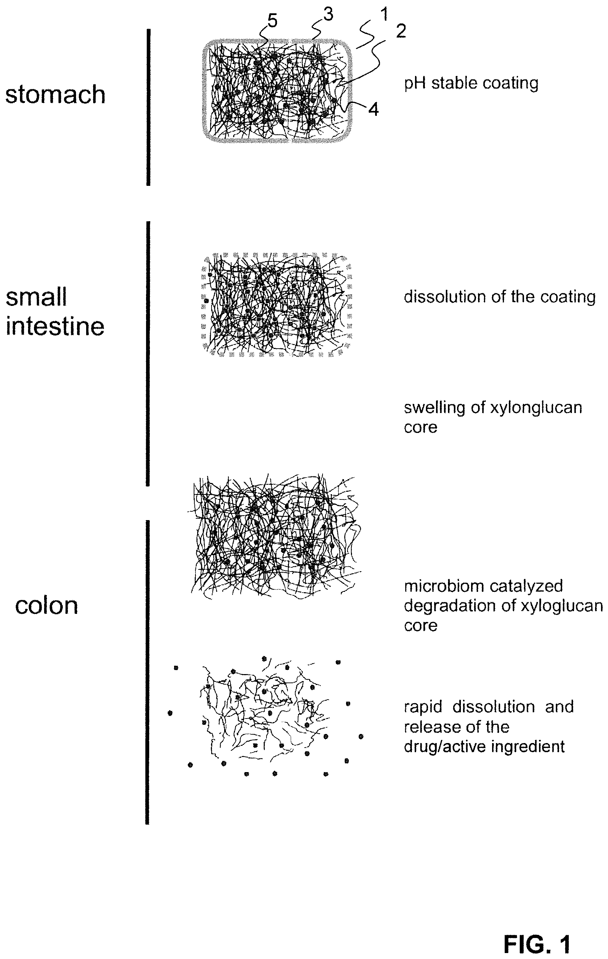

Figure 1 schematically shows the working principle of the proposed pharmaceutical dosage form 1. The dosage form 1 comprises a core 2 which is encapsulated in a shell 3. The core comprises a matrix 5, in this particular case xyloglucan, in which the active pharmaceutical ingredient 4 (API) is embedded.

The materials of the core, in particular its matrix, as well as of the shell are adapted for selective release of the API in the colon. In this respect it is to be noted that in the stomach typically there is a pH of 1.2, and the average residence time in the stomach is about two hours. Then follows the proximal small intestine again with a typical residence time of two hours and an increased pH of 6.5. This is followed by the distal small intestine with again a typical average residence time of two hours and a pH of 6.8. Only then follows the colon, first with the ascending colon followed by the descending colon, the residence time here depends on various factors, the pH is still in the range of 6.8.

The shell 3 of the proposed formulation dosage form is adapted to only dissolve significantly once the pH increases above 6.5, typically reaches a value of at least 6.8. Correspondingly the core portion of the tablet only starts to dissolve in the small intestine. However that is not yet the place where the API is to be released. To this end that xyloglucan is forming the matrix of the core. Under the physiological conditions in the small intestine the core portion now essentially without coating swells and forms a highly viscous mass but does not release the active ingredient to a significant extent. Only once this swollen matrix still containing the API enters the colon with the different micro-organisms containing enzymes digesting xyloglucan the matrix is digested and disintegrates and then also the API is released in a targeted manner to the place where it shall develop its effects.

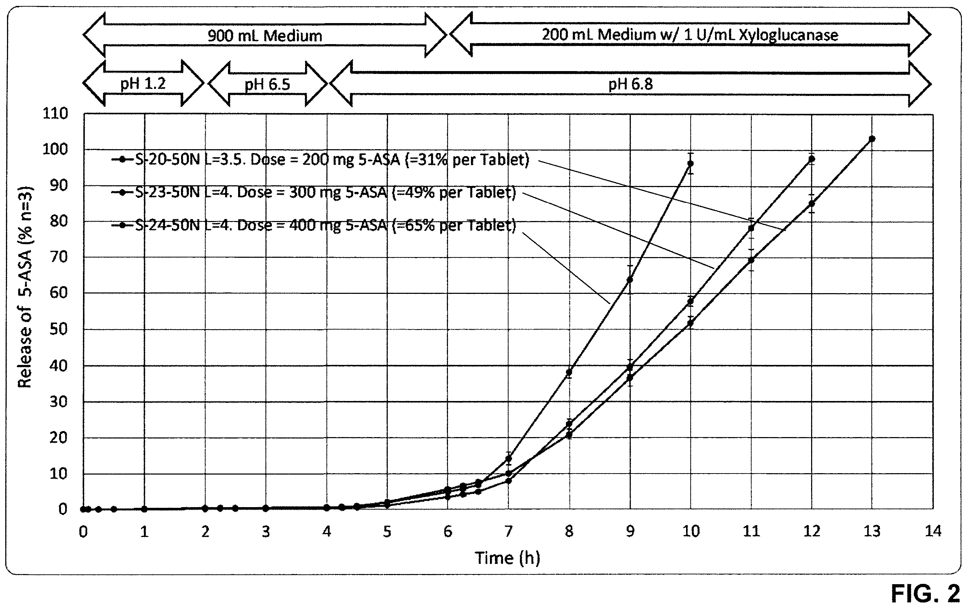

This is evidenced by the release profile illustrated in figure 2. In in vitro experiments (for the detail see further below) for the first two hours the corresponding tablet is subjected to a pH of 1.2 simulating stomach conditions. No release of the API can be detected. Subsequently for another two hours the conditions of the proximal small intestine are simulated with a pH of 6.5. Again no release of the API can be detected. Then for another two hours the conditions of the distal small intestine are simulated by increasing the pH value of 6.8 but still not changing the enzymatic surrounding. One can see that at this moment in time first small portion of the API is released, which is associated with the dissolution of the pH-dependent coating. After that time period, so after six hours counting from the start, also the enzymatic conditions are adapted to the ones as present in the colon, in particular xyloglucanase is introduced into the medium. As from this moment on, there is a sharp and targeted release of the API. As a matter of fact, the release is largely independent of the concentration of the API in the xyloglucan matrix.

**Material and methods**

**Tablet production**

A three step process was employed.

1. Granulation

Granulation was carried out either in a fluidized bed granulator or in a high shear mixer. The composition was as follows:

| | |
|---|---|
| Glyloid 3S | (93-X)% |
| 5-ASA (API) | X% |
| Polyvinylpyrrolidone (Kollidon 30) | 7% |

Three different compositions with the following values of X were used: 33.3%; 50%; 66.7%. For fluidized bed granulation with batch size of 600 g the first two ingredients were first blended in a Turbula mixer for 7 min. at 32 rpm and the third ingredient was dissolved in purified water in a concentration of 10% w/w. This solution was sprayed in the fluidized bed at a rate of 14 g/min at first that was reduced to 7.3 and then 9.7 g/min. The atomizing pressure was 1.3 to 1.5 bar. The air volume stream was at first 40 m3/h and was increased to 80 m3/h. The inlet temperature was 50°C and the product temperature was approx. 25°C throughout the liquid addition and increased to 29°C during drying. The total process duration was approx. 65 min and the residual moisture was 6.8%. The granules were passed through a 1 mm mesh screen.

For high shear granulation with batch size of 300 g all three ingredients were first blended in a Turbula mixer for 7 min at 32 rpm. Purified water was sprayed in the mixer at a rate of 6.5 g/min and an atomizing pressure of 0.12 bar. The rotation rate of the main impeller was 220 rpm and of the chopper 2200 rpm. Between 110 and 170 g of water was added yielding an increase of the power consumption of the main impeller from 82 to 91 - 93 Watt. The granules were passed through a 1 mm mesh screen, dried in a tray drier at 50°C to a residual moisture of <5% and passed again through a 0.85 mm mesh screen.

2. Tableting

Granulated compositions were blended with 0.5% Mg-stearate in a Turbula mixer for 2 min at 32 rpm. Tablets with a diameter of 12 mm, a radius of curvature of 9 mm and a diametric crushing force of 50 N were produced in a single punch eccentric tableting machine at a rate of 20 tablets per minute. The tablet weight was adjusted based on the API content of the compositions determined after granulation to between 600 and 630 mg to reach an API content of 200, 300 and 400 mg per tablet. The compression force of the upper punch was between 10 and 13 kN.

3. Coating

Tablets were coated with Eudragit FS-30-D in a drum coater with batch size of 600 g. The composition of the coating dispersion was as follows:

| | |
|---|---|
| Eudragit FS-30-D | 43% |
| Triethyl-citrate | 0.65% |
| Talc | 6.45% |
| Dye (iron III oxide) | 0.2% |
| Purified water | 49.7% |

The drum rotation speed was 20 rpm, the inlet air temperature 50°C, the product temperature 30-35°C, and the air volume stream 25-30 m3/h. The coating dispersion was sprayed at a rate of 4 g/min and an atomizing pressure of 1.3 bar. The nominal dry coating amount was L=5 mg/cm2 and the actual value was between L=3.5 and 4 mg/cm2.

Material characteristics

Xyloglucan:

Brand name: Glyloid 3S and Glyloid 2A (DSP GOKYO FOOD & CHEMICAL Co., Ltd. Osaka, Japan)
Common name: Tamarind seed polysaccharide or tamarind seed gum
Chemical substance: Xyloglucan
Gras status declaration by FDA: GRN No. 503; Substance: Tamarind seed polysaccharide; Intended Use: Use as a thickener, stabilizer, emulsifier, and gelling agent in certain food categories; Notifier: DSP GOKYO FOOD & CHEMICAL Co., Ltd.; HERBIS OSAKA 20th Floor 2-5-25 Umeda Kita-ku, Osaka, 530-0001 Japan; Date of filing: Mar 5, 2014

GRAS Notice (releasable information): 503; Date of closure: Aug 12, 2014.

| Physical-chemical properties declared by the supplier | | |
|---|---|---|
| | Glyloid 2A** | Glyloid 3S |
| Content | 80 - 99.99 % | 90 - 99.99 % |
| Glucose (inpurity) | 0.01 - 20 % | 0.01-10% |
| Water solubility | Soluble | Soluble |
| Organic solvent solubility | Not soluble | Not soluble |

(continued)

| Physical-chemical properties declared by the supplier | | |
|---|---|---|
| | Glyloid 2A** | Glyloid 3S |
| Molecular weight * | Approx. 470'000 | Approx. 470'000 |
| Physical form | White to light brown powder | White to light brown powder |
| Loss on drying * | (more than of 3S quality) | 1.1 % |
| Viscosity * | (less than of 3S quality) | 730 mPas |
| * Values in the FDA document deviate; ** not according to the invention | | |
| Physical-chemical properties determined on laboratory | | |
| | Glyloid 2A** | Glyloid 3S |
| Water solubility | Not completely soluble at room temperature for 2% w/v<br>Soluble at 90°C | Soluble at room temperature for 2% w/v |
| XRPD | Semi-amorphous (crystalline part corresponds to D-glucose) | Amorphous |
| Particle size | d(10%) = 25 $\mu$m<br>d(50%) = 60 $\mu$m<br>d(90%) = 164 $\mu$m | d(10%) = 57.7 $\mu$m<br>d(50%) = 91.4 $\mu$m<br>d(90%) = 136.7 $\mu$m |
| Viscosity | Pseudo-plastic 603.7 mPas (at 100 s$^{-1}$ 90°C) | Pseudo-plastic 721 - 852 mPas (at 100 s$^{-1}$ room temperature) |
| Molecular weight from intrinsic viscosity [$\eta$] =kM$_r$$^\alpha$ | | M$_r$ = 419'148<br><br>for k = 0.0008 $\alpha$=0.66 |
| ** not according to the invention | | |

| | |
|---|---|
| 5-ASA: | Mesalazine, also known as mesalamine or 5-aminosalicylic acid, and is an aminosalicylate anti-inflammatory drug used to treat inflammatory bowel disease, including ulcerative colitis, or inflamed anus or rectum, and to maintain remission in Crohn's disease. |
| Kollidon 30: | Polyvinylpyrrolidone, with an average molecular weight expressed in terms of the K-value as in the pharmacopoeias valid in Europe, the USA and Japan, calculated from the relative viscosity in water in the range of 27.0-32.4. |
| Eudragit FS-30-D: | is the aqueous dispersion of an anionic copolymer based on methyl acrylate, methyl methacrylate and methacrylic acid. The ratio of the free carboxyl groups to the ester groups is approx. 1:10. It is provided as an aqueous dispersion with 30 % dry substance. The dispersion contains 0.3 % Sodium Laurilsulfate Ph. Eur. / NF and 1.2 % Polysorbate 80 Ph. Eur. / NF on solid substance, as emulsifiers. Based on SEC method the weight average molar mass (Mw) is approx. 280,000 g/mole. |
| Talc | particle size: 99.5% < 75 micrometer, median 19.3 micrometer, Ph.Eur. Specific surface (BET) 3.5 m2/g, producer: Imerys Talc, Italy SpA/Luzenac Pharma. |

**Experimental Methods:**

Drug release was measured in a USP2 apparatus at 37°C with paddle rotation rate 100 rpm.

One tablet per vessel was used. A four stage test was performed with the following medium composition: In the first two hours the medium consisted of 900 mL 0.1 N HCl solution in purified water with pH 1.2.

In the following two hours the medium consisted of 900 mL 100 mM potassium phosphate monobasic adjusted to pH 6.5 with NaOH.

In the following two hours the pH of the medium was adjusted to 6.8 with NaOH.

In the last stage the medium was exchanged with 200 mL of the same phosphate buffer pH 6.8 that contained different concentrations of xyloglucanase. The latter was a microbial enzyme of Paenibacillus sp. that is specific for digestion of Xyloglucan. The unit titer is calibrated with tamarind xyloglucan.

The first test stage (pH 1.2) simulates the stomach environment while the second test stage (pH 6.5) simulates the passage through the upper small intestine. The pH 6.8 stage corresponds to the movement of the tablet to the lower small intestine and the last stage with the reduced fluid volume and the presence of microbial enzyme corresponds to the environment in the colon, where release shall take place.

**Results and discussion:**

Typical results are shown in the graphic in Fig. 3. No drug is released in the conditions reflecting the stomach and the upper small intestine, this being due to the polymeric coating.

The residence time of two hours in each of these environments is representative for the transit time of solid dosage forms in the gastrointestinal tract after a light meal as found by scintigraphy and other methods.

The coating film was designed to dissolve and be removed from the surface of the tablet at pH 6.8. This results in a modest release of API that did not exceed 10% in two hours. The rate of drug release was markedly accelerated in the presence of the microbial enzyme in a concentration dependent fashion providing the proof of principle of controlled and position-triggered drug release by the developed delivery system.

After dissolution of the polymeric coating, release is inhibited by the Xyloglucan that does not allow the tablet to disintegrate forming a highly viscous gel or gluey mass instead which acts as diffusion barrier. Although pH values as high as 7.2 have been reported for the distal small intestine the coating is deliberately designed to dissolve at lower pH, the reason being that pH values and residence times in the intestine may fluctuate and exhibit inter-individual variability, and that the pH in the ascending colon can be again <7. Hence, if a film coating does not dissolve in the small intestine in a timely fashion, the tablet may be defecated intact as observed already with other experimental systems.

For the present delivery system, an early, i.e. at low pH, removal of the coating does not result in excessive release of drug in the small intestine and thus an undesirable loss of API for local action in the colon as this is prevented by the Xyloglucan matrix. Upon entering the colon the action of localized microbiome-specific enzymes ensures a rapid drug release. Hence the synergistic overlap of two control mechanisms provides a highly targeted delivery to the colon. The employed enzyme concentrations correspond to those reported and reasonably expected to be found in the human large intestine.

The property of Xyloglucan to slow down drug release is demonstrated in Figure 4. Depending on the porosity of un-coated tablets release of the API can be adapted to take much longer than 24 hours while the release process follows approximately zero-order kinetics. The accelerating effect of enzymatic degradation of Xyloglucan on drug release is shown on Figure 3.

Figure 5 shows the drug release from tables as a function of time and pH for various modifications, for no coating (6) and with coating (7-10) situations. All cores of the tablets had a crushing force of 50 N. The coating thickness defined as coating mass per square centimeter of tablet surface increased from $2 \, mg/cm^2$ to $3.4. \, mg/cm^2$ to $4.9 \, mg/cm^2$ to $6.8 \, mg/cm^2$ for curves 7 to 10, respectively. The results show the optimal coating thickness for effective enteric coating and timely dissolution of the coating. The results also show that no burst release and therefore better release control takes place after the coating is dissolved at pH 6.8 contrary to the absence of a coating at the same pH (pH 7), shown in Figure 4, underlining the synergy effect between coating and xyloglucan.

**Distinction from prior art:**

As for the distinction from the above-mentioned Yoo publication the following is to be noted: The results of the Yoo publication prove that the manufactured product (beads) do not work like the tablets proposed here. In Figure 5 of Yoo the release of the active substance is measured first in the simulated stomach medium pH 1.2 and then in the simulated intestinal medium pH 7.4. The coated beads of Yoo show a release of about 10% in the first 2 hours at pH 1.2 and about 50% in the next 2 hours at pH 7.4. The formulation as described here shows a release of 0% at pH 1.2 after 2 hours, a release of 0% at pH 6.5 after 2 hours and a release of about 5% at pH 6.8 after a further 2 hours. Since the goal is to have as little release as possible under intestinal conditions, the product in Yoo by far does not meet our requirements.

Furthermore the experiment from Yoo was replicated as follows using native xyloglucan as used here instead of the degalactosylated type of Yoo:

- a 2% xyloglucan solution (quality 3S as in the tests here) in water was prepared at 4°C while stirring with propeller stirrer during 24 hours.
- plant oil was heated at 40°C and at 80°C under magnetic stirring.
- 1 mL of xyloglucan solution was dripped through a syringe with needle (ID 0.71 mm corresponding to 22G) to the oil.

The result has been documented photographically.

At both temperatures, drops with a size of about 2 to 2.5 mm are formed at the beginning. At 40°C the drops flow together to worms after a few minutes.

At 80°C air bubbles have formed in the droplets, the droplets have risen to the surface and flowed together during filtering.

Based on this the following interpretation has to be drawn: Native xyloglucan (not de-galactosylated) does not gel. De-galactosylated xyloglucan forms gels. The temperature of gel formation depends on the degree of de galactolysis. This is confirmed by independent work (e.g. above mentioned Brun-Graeppi publication). The xyloglucan of Yoo has a 44% galactose removal and gels at 40°C. Native xyloglucan shows nothing up to 60°C (Fig. 3 of Brun-Graeppi). We have gone up to 80°C but also no gelation seen, gelation means a solidification of the drops. This prevents the droplets from flowing together. In Yoo the droplets are cured for 30 minutes at 40°C, filtered, washed with acetone and dehydrated with successive water/ethanol mixtures. This was not possible with the drops using the native xyloglucan because they flowed together and coalesced. The difference lies clearly in the de-galactosylation and the associated gelation, making the Yoo method impossible using native xyloglucan.

The small beads of Yoo are fundamentally different from the compressed tables given here at least for the following reasons: The beads from Yoo, *not coated,* with a drug load of 27.77% (Charge XGID 100, Table 1) show a drug release of above 70% in 2 hours at pH 7.4 (Fig. 2a). Our tablets with a drug load of 30% (comparable to the beads) show a drug release between 10 and 35% at pH 7 within 2 hours (also comparable to the beads, Fig. 4 given here) depending on the strength of the compression. Strongly compressed tablets with a high strength (134 N crushing force) have a porosity of 14.8% i.e. a relative density of 0.852 (space filling of 85.2%) and show a release in 2 hours of almost 10%. Slightly compressed tablets (crushing force = 30 N, relative density = 0.713) show a release in 2 hours of about 35%. The results clearly demonstrate the great influence of the volume and/or density of the tablets and the compression process on the release (without coating). The density of the beads must be significantly below 70% due to the manufacturing method (syringe method as given above). One starts with a 2% xyloglucan solution to which one adds a maximum of 2% active ingredient. With this, one cannot reach a solid content of 70 V-% in the beads at the end. This is visible from the very different amount of release between beads and tablets in general.

The release *with coating* also works differently with the beads of Yoo than with our tablets. Fig. 5 of Yoo shows release after 2 hours pH 1.2 (stomach conditions) with coating almost 10% and after further 2 hours at pH 7.4 (small intestine conditions) release of total 50%. In the same duration under the same conditions without coating the release from the beads is about 65%. This means firstly that the coating makes a relatively small difference (50 vs. 65%) and secondly that the goal of releasing as little active substance as possible in the small intestine is not achieved. In our tablets (Fig. 3 given here) we have a release of 0% at pH 1.2 after 2 hours, a release of 0% after 2 hours at pH 6.5 (upper conditions of the small intestine) and a release of about 5% after a further 2 hours at pH 6.8 (conditions of the small intestine). Without coating under the same conditions we have a release of about 32% (figure 5 given here). This means, firstly, that the coating makes up a great deal (5 vs. 32%) and, secondly, that we achieve the goal of the lowest possible release in the small intestine (during a total of 6 hours).

In addition, the presence of the coating influences the release after the coating is dissolved and removed, i.e. at pH 6.8. If one compares the release in Fig. 4 at pH 7 with the release at pH 6.8 in further Fig. 5, one can see that in the case of a coating the burst effect (sudden increase) is omitted at the beginning while afterwards the release rate in the case of the coating is somewhat higher (than without coating). The latter can also be seen in the presence of the microbial enzyme. This has to do with the fact that the inside of the tablet is still intact in the first 4 to 6 hours during the coating, absorbs water and the xyloglucan starts to swell which influences the subsequent release. So there is a synergistic effect between xyloglucan core and coating which influences the release.

In contrast, using the formulation given here, tablets (several mm diameter) of core and API are prepared and the core is later coated with an enteric film. The release of the API is as follows: No or only minor release is expected and observed at low pH (gastric passage). Upon neutralization (entry in the small intestine) the enteric coating dissolves. Water will now wet the tablet and water will diffuse into the tablet. In the outer surface of the tablet core xyloglucan (solid, no air) will form a highly viscous mass impeding release of API and slowing down water intrusion. Consequently, there will be only a minor release of the API for several hours ("delayed release").

The polysaccharide xyloglucan is not degraded by human digestive enzymes. Instead, xyloglucan and other plant cell wall derived polysaccharides are degraded and metabolized by the colonic microbiome. We could indeed show that in the presence of xyloglucanase (the enzyme which initiates degradation of xyloglucan) the API release is accelerated which is probably caused by the accelerated erosion of the xyloglucan matrix by the enzymatic degradation. The specific degradation of xyloglucan by the colonic microbiome constitutes the second control mechanism of our technology.

LIST OF REFERENCE SIGNS

| 1 | pharmaceutical formulation dosage form, tablet | 4 | active pharmaceutical ingredient |
|---|---|---|---|
| 2 | core | 5 | matrix, xyloglucan |
| 3 | shell | 6 | S-20-50N total mass 631 mg |
|  | no coating | 9 | S-19-50N Eudragit FS30, L=4.9 Total mass 671 mg with coating |
| 7 | S-20-50N Eudragit FS30, L=2 Total mass 639 mg with coating |  |  |
| 8 | S-20-50N Eudragit FS30, L=3.5 Total mass 651 mg with coating | 10 | S-20-50N Eudragit FS30, L=6.8 Total mass 661 mg with coating |

**Claims**

1. Pharmaceutical formulation dosage form (1) with a core (2) encapsulated by at least one shell (3) and comprising at least one active pharmaceutical ingredient (4),

   wherein the at least one active pharmaceutical ingredient (4) is embedded in said core (2) or forming said core (2) of the pharmaceutical formulation dosage form (1),
   wherein said shell (3) comprises a pH-responsive coating,
   and wherein at least one of said core (2) and said shell (3) is based on native, highly purified cold-water soluble type xyloglucan.

2. Pharmaceutical formulation dosage form (1) according to claim 1, wherein the at least one active pharmaceutical ingredient (4) is embedded in said core (2) of the pharmaceutical formulation dosage form (1) in that said core (2) is formed by a matrix based on said native, highly purified cold-water soluble type xyloglucan (5) containing said active pharmaceutical ingredient (4),

   and/or wherein the core is a single solid compressed core with a relative density of at least 0.7, preferably of at least 0.75 or at least 0.8;
   and/or wherein the core and/or the whole pharmaceutical formulation dosage form has an average diameter in the direction of the smallest diameter of at least 3 mm, preferably at least 4 mm, more preferably at least 4.5 or 5 mm;
   and/or wherein the core and/or the whole pharmaceutical formulation dosage form has a crushing force of at least 25N, preferably of at least 40N, more preferably of at least 100N.

3. Pharmaceutical formulation dosage form (1) according to any of the preceding claims, wherein said shell (3) comprises at least one outer layer in the form of a pH responsive coating, based on said native, highly purified cold-water soluble type xyloglucan (5) or free from xyloglucan (5), as well as at least one inner layer based on said native, highly purified cold-water soluble type xyloglucan (5) if the outer layer is free from xyloglucan (5).

4. Pharmaceutical formulation dosage form (1) according to any of the preceding claims, wherein it is adapted for oral administration and for targeted release of the active pharmaceutical ingredient in the colon, and wherein said shell (3) comprises at least one or consists of a pH-responsive coating dissolving only at a pH of more than 6.5, preferably of at least 6.7, more preferably of at least 6.8.

5. Pharmaceutical formulation dosage form (1) according to any of the preceding claims, wherein said shell (3), in particular the at least one pH responsive coating thereof, is based on a synthetic polymer and/or a biopolymer or a mixture thereof, preferably based on an anionic acrylate copolymer, preferably on an anionic copolymer based on methyl acrylate, methyl methacrylate and methacrylic acid, wherein preferably the ratio of the free carboxyl groups to the ester groups is in the range of 1:5 - 1:10, preferably in the range of 1:10, wherein preferably the anionic acrylate copolymer has a weight average molar mass (Mw) in the range of 200'000-400'000 g/mole, preferably in the range of 250°000-300°000 g/mole,

6. Pharmaceutical formulation dosage form (1) according to any of the preceding claims, wherein said shell (3), in particular the at least one pH responsive coating thereof, consists of a mixture of an anionic acrylate copolymer, preferably on an anionic copolymer based on methyl acrylate, methyl methacrylate and methacrylic acid, wherein preferably the ratio of the free carboxyl groups to the ester groups is in the range of 1:5 - 1:10, preferably in the range of 1:10, wherein preferably the anionic acrylate copolymer has a weight average molar mass (Mw) in the range of 200'000-400'000 g/mole, preferably in the range of 250°000-300°000 g/mole, with further additives in a proportion of less than 25%, said further additives preferably being selected from the group consisting of polyoxyethylene and derivatives thereof, anionic surfactants, in particular sodium laurylsulfate, talc, dye, in particular iron(III)oxide, stabilizers, in particular triethyl citrate,

7. Pharmaceutical formulation dosage form (1) according to any of the preceding claims, wherein the dry coating amount of the at least one pH responsive coating or of the whole shell (3) is in the range of 1-10, preferably in the range of 2.5-6 mg/cm2, or wherein there is provided only one single encapsulating pH responsive coating (3) forming said shell (3).

8. Pharmaceutical formulation dosage form (1) according to any of the preceding claims, wherein said matrix of the core (2) essentially or completely consists of said native, highly purified cold-water soluble type xyloglucan (5), wherein preferably said native, highly purified cold-water soluble type xyloglucan (5) is obtained from Tamarindus indica seeds and/or is cold water soluble and/or is amorphous,

and/or wherein the native, highly purified cold-water soluble type xyloglucan used as starting material has a particle size (d50%) of at least 70 $\mu$m, preferably in the range of 70-150 $\mu$m, more preferably in the range of 80-110 $\mu$m, and/or wherein the native, highly purified cold-water soluble type xyloglucan has a weight average molar mass (Mw) in the range of 400,000-500,000 g/mol.

9. Pharmaceutical formulation dosage form (1) according to any of the preceding claims, wherein the core (2) consists of

(A) 25 - 90%, preferably 40 - 90% by weight of said native, highly purified cold-water soluble type xyloglucan;
(B) 10 - 60% by weight of at least one active pharmaceutical ingredient;
(C) 0 - 20% by weight, preferably 5 - 10% by weight of one or more pharmaceutically acceptable excipients selected from the group consisting of a diluent, a binder, an anti-adherent, a lubricant, a glidant and a combination thereof, wherein preferably the pharmaceutically acceptable excipient essentially consists of a binder or a binder and an anti-adherent, in particular the binder being selected as PVP and the anti-adherent as magnesium stearate
or wherein the core (2) consists of granules consisting of

(A) 25 - 90%, preferably 40-90% by weight of said native, highly purified cold-water soluble type xyloglucan;
(B) 10-60% by weight of at least one active pharmaceutical ingredient;
(C) 0-20% by weight, preferably 5-10% by weight of one or more pharmaceutically acceptable excipients selected from the group consisting of a diluent, a binder, a lubricant, a glidant and a combination thereof, wherein preferably the pharmaceutically acceptable excipient essentially consists of a binder, in particular the binder being selected as PVP,

which granules are compacted to form a core (2) before applying the shell, wherein preferably before compacting the granules are blended with an anti-adherent, preferably in the form of magnesium stearate.

10. Pharmaceutical formulation dosage form (1) according to any of the preceding claims, wherein the weight ratio of the matrix of the core (2) to the at least one active pharmaceutical ingredient is at least 1:2, preferably at least 1:1, more preferably at least 2:1.

11. Pharmaceutical formulation dosage form (1) according to any of the preceding claims suitable for the purpose of establishing, re-establishing and/or modifying the balance of the microbiome population in the colon or the physiology of the lower gastrointestinal tract.

12. Pharmaceutical formulation dosage form (1) according to any of the preceding claims for use as immunomodulation or immunosuppression or for the treatment of at least one of the following conditions: inflammatory bowel disease, in particular ulcerative colitis and/or Crohn's disease, Clostridium difficile infection, colon cancer, post colon surgical treatment.

13. Pharmaceutical formulation dosage form (1) according to any of the preceding claims, wherein the active pharmaceutical ingredient is selected from the group consisting of: mesalazine, budesonide, capecitabine, fluorouracil, irinotecan, oxaliplatin, UFT, cetuximab, panitumumab, immunomodulatory or immunosuppressive ingredients including immunosuppressive glucocorticoids, immunosuppressive cytostatics, immunosuppressive (poly- or monoclonal) antibodies, immunosuppressive drugs acting on immunophilins, interleukins, cytokines, chemokines, immunomodulatory imide drug, including in particular tacrolimus, cyclosporin or materials suitable for the purpose of establishing, reestablishing and/or modifying the balance of the microbiome population in the colon or compounds which have a beneficial effect on the physiology of the lower gastrointestinal tract, or combinations thereof.

14. Pharmaceutical formulation dosage form (1) according to any of the preceding claims suitable for oral administration, wherein it is administered orally at least once a day, preferably twice a day, over a time span of at least one week, preferably at least two weeks, or at least two months or at least 1 year or even life-long.

15. Method for making a pharmaceutical formulation dosage form according to any of the preceding claims, wherein in a first step said native, highly purified cold-water soluble type xyloglucan, at least one active pharmaceutical ingredient, as well as if needed one or more pharmaceutically acceptable excipients are mixed and then compacted to form the core or mixed and treated to form granules, preferably compressed to a single core with a relative density of at least 0.7, preferably of at least 0.75 or at least 0.8 and/or with an average diameter in the direction of the smallest diameter of at least 3 mm, preferably at least 4 mm, more preferably at least 4.5 or 5 mm, which are subsequently, if needed by first mixing the granules with a further treatment agent, compacted to form the core, wherein the mixing in both cases can take place using preferably a fluidised bed granulator or high shear mixer,
and the core is subsequently coated in a second step with at least one coating forming a shell, wherein preferably the coating formulation is provided as a dispersion and is applied further preferably in a drum coater.

## Patentansprüche

1. Pharmazeutische Darreichungsform (1) mit einem Kern (2), der von mindestens einer Hülle (3) umgeben ist und mindestens einen pharmazeutischen Wirkstoff (4) enthält,

    wobei der mindestens eine pharmazeutische Wirkstoff (4) in den Kern (2) eingebettet ist oder den Kern (2) der pharmazeutischen Formulierung in Dosierungsform (1) bildet,
    wobei die Hülle (3) eine pH-empfindliche Beschichtung umfasst,
    und wobei der Kern (2), die Hülle (3), oder beide, auf nativem, hochgereinigtem, kaltwasserlöslichem Xyloglucan basiert.

2. Pharmazeutische Darreichungsform (1) nach Anspruch 1, wobei der mindestens eine pharmazeutische Wirkstoff (4) in den Kern (2) der pharmazeutischen Darreichungsform (1) eingebettet ist, indem der Kern (2) durch eine Matrix auf Basis des nativen, hochgereinigten, kaltwasserlöslichen Xyloglucans (5) gebildet ist, das den pharmazeutischen Wirkstoff (4) enthält,

    und/oder wobei der Kern ein einzelner fester komprimierter Kern mit einer relativen Dichte von mindestens 0,7, vorzugsweise von mindestens 0,75 oder mindestens 0,8 ist;
    und/oder wobei der Kern und/oder die gesamte pharmazeutische Darreichungsform einen durchschnittlichen Durchmesser in Richtung des kleinsten Durchmessers von mindestens 3 mm, vorzugsweise mindestens 4 mm, noch bevorzugter mindestens 4,5 oder 5 mm aufweist;
    und/oder wobei der Kern und/oder die gesamte pharmazeutische Formulierung eine Zerkleinerungskraft von mindestens 25 N, vorzugsweise von mindestens 40 N, noch bevorzugter von mindestens 100 N aufweist.

3. Pharmazeutische Darreichungsform (1) nach einem der vorstehenden Ansprüche, wobei die Hülle (3) mindestens eine äussere Schicht in Form einer pH-empfindlichen Beschichtung auf der Basis des nativen, hochgereinigten, kaltwasserlöslichen Xyloglucans (5) oder frei von Xyloglucan (5) umfasst, sowie mindestens eine Innenschicht auf Basis des nativen, hochgereinigten, kaltwasserlöslichen Xyloglucans (5), wenn die Aussenschicht frei von Xyloglucan (5) ist.

4. Pharmazeutische Darreichungsform (1) gemäss einem der vorstehenden Ansprüche, wobei sie für die orale Verabreichung und für die gezielte Freisetzung des pharmazeutischen Wirkstoffs im Dickdarm geeignet ist und wobei die Hülle (3) mindestens eine pH-empfindliche Beschichtung umfasst oder aus einer solchen besteht, die sich nur bei einem pH-Wert von mehr als 6,5, vorzugsweise von mindestens 6,7, noch bevorzugter von mindestens 6,8, auflöst.

5. Pharmazeutische Darreichungsform (1) nach einem der vorstehenden Ansprüche, wobei die Hülle (3), insbesondere die mindestens eine pH-empfindliche Beschichtung davon, auf einem synthetischen Polymer und/oder einem Biopolymer oder einer Mischung davon basiert, vorzugsweise auf einem anionischen AcrylatCopolymer, vorzugsweise auf einem anionischen Copolymer auf Basis von Methylacrylat, Methylmethacrylat und Methacrylsäure, wobei vorzugsweise das Verhältnis der freien Carboxylgruppen zu den Estergruppen im Bereich von 1:5 - 1:10, vorzugsweise im Bereich von 1:10 liegt, wobei das anionische Acrylatcopolymer vorzugsweise eine gewichtsmittlere Molmasse (Mw) im Bereich von 200 000-400 000 g/mol, vorzugsweise im Bereich von 250 000-300 000 g/mol, aufweist,

6. Pharmazeutische Darreichungsform (1) nach einem der vorstehenden Ansprüche, wobei die Hülle (3), insbesondere die mindestens eine pH-empfindliche Beschichtung davon, aus einer Mischung eines anionischen Acrylatcopolymers, vorzugsweise eines anionischen Copolymers auf Basis von Methylacrylat, Methylmethacrylat und Methacrylsäure, wobei vorzugsweise das Verhältnis der freien Carboxylgruppen zu den Estergruppen im Bereich von 1:5 bis 1:10, vorzugsweise im Bereich von 1:10 liegt, wobei vorzugsweise das anionische Acrylatcopolymer eine gewichtsmittlere Molmasse (Mw) im Bereich von 200'000-400 000 g/mol, vorzugsweise im Bereich von 250 000 - 300 000 g/mol, aufweist, mit weiteren Additiven in einem Anteil von weniger als 25 %, wobei die weiteren Additive vorzugsweise aus der Gruppe ausgewählt sind, die aus Polyoxyethylen und Derivaten davon, anionischen Tensiden, insbesondere Natriumlaurylsulfat, Talk, Farbstoff, insbesondere Eisen(III)-oxid, Stabilisatoren, insbesondere Triethylcitrat,

7. Pharmazeutische Darreichungsform (1) nach einem der vorstehenden Ansprüche, wobei die Trockenbeschichtungsmenge der mindestens einen pH-empfindlichen Beschichtung oder der gesamten Hülle (3) im Bereich von 1 bis 10, vorzugsweise im Bereich von 2,5 bis 6 mg/cm2 liegt,
oder wobei nur eine einzige pH-empfindliche Beschichtung (3) vorgesehen ist, die die Hülle (3) bildet.

8. Pharmazeutische Darreichungsform (1) nach einem der vorstehenden Ansprüche, wobei die Matrix des Kerns (2) im Wesentlichen oder vollständig aus dem nativen, hochgereinigten, kaltwasserlöslichen Xyloglucan (5) besteht, wobei das native, hochgereinigte, kaltwasserlösliche Xyloglucan (5) vorzugsweise aus Tamarindus indica-Samen gewonnen wird und/oder kaltwasserlöslich und/oder amorph ist,

und/oder wobei das als Ausgangsmaterial verwendete native, hochgereinigte, kaltwasserlösliche Xyloglucan eine Partikelgrösse (d50 %) von mindestens 70 $\mu$m, vorzugsweise im Bereich von 70 bis 150 $\mu$m, noch bevorzugter im Bereich von 80 bis 110 $\mu$m, aufweist.
und/oder wobei das native, hochgereinigte, kaltwasserlösliche Xyloglucan eine gewichtsmittlere Molmasse (Mw) im Bereich von 400.000 bis 500.000 g/mol aufweist.

9. Pharmazeutische Darreichungsform (1) gemäss einem der vorstehenden Ansprüche, wobei der Kern (2) besteht aus

(A) 25 bis 90 Gew.-%, vorzugsweise 40 bis 90 Gew.-%, des nativen, hochgereinigten, kaltwasserlöslichen Xyloglucans;
(B) 10 bis 60 Gew.-% mindestens eines pharmazeutischen Wirkstoffs;
(C) 0 bis 20 Gew.-%, vorzugsweise 5 bis 10 Gew.-%, eines oder mehrerer pharmazeutisch verträglicher Hilfsstoffe, ausgewählt aus der Gruppe bestehend aus einem Verdünnungsmittel, einem Bindemittel, einem Antihaftmittel, einem Gleitmittel, einem Gleitmittel und einer Kombination davon, wobei der pharmazeutisch verträgliche Hilfsstoff vorzugsweise im Wesentlichen aus einem Bindemittel oder einem Bindemittel und einem Antihaftmittel besteht, wobei insbesondere das Bindemittel als PVP und das Antihaftmittel als Magnesiumstearat ausgewählt ist
oder wobei der Kern (2) aus Granulaten besteht, die aus

(A) 25 bis 90 Gew.-%, vorzugsweise 40 bis 90 Gew.-%, des nativen, hochgereinigten, kaltwasserlöslichen Xyloglucans;
(B) 10 bis 60 Gew.-% mindestens eines pharmazeutisch wirksamen Bestandteils;
(C) 0 bis 20 Gew.-%, vorzugsweise 5 bis 10 Gew.-% eines oder mehrerer pharmazeutisch verträglicher Hilfsstoffe, ausgewählt aus der Gruppe bestehend aus einem Verdünnungsmittel, einem Bindemittel, einem Gleitmittel, einem Fliesshilfsmittel und einer Kombination davon, wobei der pharmazeutisch verträgliche Hilfsstoff vorzugsweise im Wesentlichen aus einem Bindemittel besteht, insbesondere wobei das Bindemittel als PVP ausgewählt ist,

wobei die Granulate vor dem Aufbringen der Hülle zu einem Kern (2) verdichtet werden, wobei die Granulate vorzugsweise vor dem Verdichten mit einem Antihaftmittel, vorzugsweise in Form von Magnesiumstearat, gemischt werden.

**10.** Pharmazeutische Darreichungsform (1) nach einem der vorstehenden Ansprüche, wobei das Gewichtsverhältnis der Matrix des Kerns (2) zu dem mindestens einen pharmazeutischen Wirkstoff mindestens 1:2, vorzugsweise mindestens 1:1, noch bevorzugter mindestens 2:1 beträgt.

**11.** Pharmazeutische Darreichungsform (1) gemäss einem der vorstehenden Ansprüche, die zum Aufbau, Wiederaufbau und/oder zur Veränderung des Gleichgewichts der Mikrobiom-Population im Dickdarm oder der Physiologie des unteren Magen-Darm-Trakts geeignet ist.

**12.** Pharmazeutische Formulierung (1) gemäss einem der vorstehenden Ansprüche zur Verwendung als Immunmodulation oder Immunsuppression oder zur Behandlung mindestens einer der folgenden Erkrankungen: entzündliche Darmerkrankungen, insbesondere Colitis ulcerosa und/oder Morbus Crohn, Clostridium-difficile-Infektion, Darmkrebs, Behandlung nach einer Darmoperation.

**13.** Pharmazeutische Darreichungsform (1) gemäss einem der vorstehenden Ansprüche, wobei der pharmazeutische Wirkstoff aus der Gruppe ausgewählt ist, die besteht aus: Mesalazin, Budesonid, Capecitabin, Fluorouracil, Irinotecan, Oxaliplatin, UFT, Cetuximab, Panitumumab, immunmodulatorischen oder immunsuppressiven Inhaltsstoffen, einschliesslich immunsuppressiver Glukokortikoide, immunsuppressiver Zytostatika, immunsuppressiver (poly- oder monoklonaler) Antikörper, immunsuppressiver Arzneimittel, die auf Immunophiline wirken, Interleukine, Zytokinen, Chemokinen, immunmodulatorischen Imid-Arzneimitteln, insbesondere Tacrolimus, Cyclosporin oder Materialien, die zum Aufbau, Wiederherstellung und/oder zur Modifizierung des Gleichgewichts der Mikrobiom-Population im Dickdarm geeignet sind, oder Verbindungen, die eine positive Wirkung auf die Physiologie des unteren Magen-Darm-Trakts haben, oder Kombinationen davon.

**14.** Pharmazeutische Darreichungsform (1) gemäss einem der vorstehenden Ansprüche, die zur oralen Verabreichung geeignet ist, wobei sie mindestens einmal täglich, vorzugsweise zweimal täglich, über einen Zeitraum von mindestens einer Woche, vorzugsweise mindestens zwei Wochen, oder mindestens zwei Monaten oder mindestens einem Jahr oder sogar lebenslang oral verabreicht wird.

**15.** Verfahren zur Herstellung einer pharmazeutischen Darreichungsform gemäss einem der vorstehenden Ansprüche, wobei in einem ersten Schritt das native, hochgereinigte, kaltwasserlösliche Xyloglucan mindestens ein pharmazeutisch wirksamer Bestandteil sowie, falls erforderlich, ein oder mehrere pharmazeutisch verträgliche Hilfsstoffe gemischt und dann zu einem Kern verdichtet oder gemischt und zu Granulat verarbeitet werden, vorzugsweise zu einem einzigen Kern mit einer relativen Dichte von mindestens 0,7, vorzugsweise von mindestens 0,75 oder mindestens 0,8 und/oder mit einem durchschnittlichen Durchmesser in Richtung des kleinsten Durchmessers von mindestens 3 mm, vorzugsweise mindestens 4 mm, noch bevorzugter mindestens 4,5 oder 5 mm, die anschliessend, falls erforderlich, durch erstes Mischen der Granulate mit einem weiteren Behandlungsmittel zu einem Kern verdichtet werden, wobei das Mischen in beiden Fällen vorzugsweise unter Verwendung eines fluidised bed Granulators oder eines Hochscher-Mischers erfolgen kann,
und der Kern anschliessend in einem zweiten Schritt mit mindestens einer eine Hülle bildenden Beschichtung beschichtet wird, wobei die Beschichtungsformulierung vorzugsweise als Dispersion bereitgestellt und weiter bevorzugt in einem Trommelbeschichter aufgebracht wird.

**Revendications**

**1.** Forme posologique de formulation pharmaceutique (1) comprenant un noyau (2) encapsulé par au moins une enveloppe (3) et contenant au moins un ingrédient pharmaceutique actif (4),

dans laquelle ledit au moins un ingrédient pharmaceutique actif (4) est incorporé dans ledit noyau (2) ou forme ledit noyau (2) de la forme posologique pharmaceutique (1),
dans laquelle ladite enveloppe (3) comprend un enrobage sensible au pH,
et dans laquelle au moins l'un dudit noyau (2) et de ladite enveloppe (3) est à base de xyloglucane natif, hautement purifié et soluble dans l'eau froide.

**2.** Forme posologique de formulation pharmaceutique (1) selon la revendication 1, dans laquelle le au moins un ingrédient pharmaceutique actif (4) est incorporé dans ledit noyau (2) de la forme posologique de formulation pharmaceutique (1) en ce que ledit noyau (2) est formé par une matrice à base dudit xyloglucane de type soluble dans l'eau froide, natif et hautement purifié (5) contenant ledit ingrédient pharmaceutique actif (4),

et/ou dans laquelle le noyau est un noyau solide comprimé unique ayant une densité relative d'au moins 0,7, de préférence d'au moins 0,75 ou d'au moins 0,8 ;

et/ou dans lequel le noyau et/ou l'ensemble de la forme posologique de la formulation pharmaceutique a un diamètre moyen dans la direction du plus petit diamètre d'au moins 3 mm, de préférence d'au moins 4 mm, plus préférablement d'au moins 4,5 ou 5 mm ;

et/ou dans lequel le noyau et/ou l'ensemble de la forme posologique de la formulation pharmaceutique a une force de broyage d'au moins 25 N, de préférence d'au moins 40 N, plus préférablement d'au moins 100 N.

3. Forme posologique de formulation pharmaceutique (1) selon l'une quelconque des revendications précédentes, dans laquelle ladite enveloppe (3) comprend au moins une couche externe sous la forme d'un revêtement sensible au pH, à base dudit xyloglucane (5) natif, hautement purifié, soluble dans l'eau froide, ou exempt de xyloglucane (5), ainsi qu'au moins une couche interne à base dudit xyloglucane (5) natif, hautement purifié et soluble dans l'eau froide si la couche externe est exempte de xyloglucane (5).

4. Forme posologique de formulation pharmaceutique (1) selon l'une quelconque des revendications précédentes, dans laquelle elle est adaptée à une administration orale et à une libération ciblée de l'ingrédient pharmaceutique actif dans le côlon, et dans laquelle ladite enveloppe (3) comprend au moins un ou consiste en un revêtement sensible au pH se dissolvant uniquement à un pH supérieur à 6,5, de préférence d'au moins 6,7, plus préférablement d'au moins 6,8.

5. Forme posologique de formulation pharmaceutique (1) selon l'une quelconque des revendications précédentes, dans laquelle ladite enveloppe (3), en particulier au moins un revêtement sensible au pH de celle-ci, est à base d'un polymère synthétique et/ou d'un biopolymère ou d'un mélange de ceux-ci, de préférence à base d'un copolymère acrylate anionique, de préférence à base d'un copolymère anionique à base d'acrylate de méthyle, méthacrylate de méthyle et acide méthacrylique, dans lequel de préférence le rapport des groupes carboxyle libres aux groupes ester est compris dans la plage de 1:5 à 1:10, de préférence dans la plage de 1:10, dans lequel, de préférence, le copolymère acrylate anionique a une masse molaire moyenne en poids (Mw) comprise dans la plage de 200 000 à 400 000 g/mole, de préférence dans la plage de 250 000 à 300 000 g/mole,

6. Forme posologique de formulation pharmaceutique (1) selon l'une quelconque des revendications précédentes, dans laquelle ladite enveloppe (3), en particulier au moins un revêtement sensible au pH de celle-ci, est constituée d'un mélange d'un copolymère acrylate anionique, de préférence un copolymère anionique à base d'acrylate de méthyle, méthacrylate de méthyle et acide méthacrylique, dans lequel de préférence le rapport des groupes carboxyle libres aux groupes ester est compris dans la plage de 1:5 à 1:10, de préférence dans la plage de 1:10, dans lequel de préférence le copolymère acrylate anionique a une masse molaire moyenne en poids (Mw) comprise dans la plage de 200 000 à 400 000 g/mole, de préférence comprise entre 250 000 et 300 000 g/mole, avec d'autres additifs dans une proportion inférieure à 25 %, lesdits autres additifs étant de préférence choisis dans le groupe constitué par le polyoxyéthylène et ses dérivés, les tensioactifs anioniques, en particulier le laurylsulfate de sodium, du talc, des colorants, en particulier l'oxyde de fer (III), des stabilisants, en particulier le citrate de triéthyle,

7. Forme posologique de formulation pharmaceutique (1) selon l'une quelconque des revendications précédentes, dans laquelle la quantité de revêtement sec d'au moins un revêtement sensible au pH ou de l'ensemble de l'enveloppe (3) est comprise entre 1 et 10, de préférence entre 2,5 et 6 mg/cm2, ou dans laquelle il n'est prévu qu'une seule d'enrobage sensible au pH (3) formant ladite enveloppe (3).

8. Forme posologique de formulation pharmaceutique (1) selon l'une quelconque des revendications précédentes, dans laquelle ladite matrice du noyau (2) est essentiellement ou complètement constituée dudit xyloglucane de type soluble dans l'eau froide, natif et hautement purifié (5), dans laquelle, de préférence, ledit xyloglucane natif, hautement purifié, soluble dans l'eau froide (5) est obtenu à partir de graines de Tamarindus indica et/ou est soluble dans l'eau froide et/ou est amorphe,

et/ou dans laquelle le xyloglucane de type natif, hautement purifié et soluble dans l'eau froide utilisé comme matière première a une taille de particules (d50 %) d'au moins 70 $\mu$m, de préférence comprise entre 70 et 150 $\mu$m, de préférence encore comprise entre 80 et 110 $\mu$m,

et/ou dans lequel le xyloglucane natif hautement purifié soluble dans l'eau froide a une masse molaire moyenne en poids (Mw) comprise entre 400 000 et 500 000 g/mol.

9. Forme posologique de formulation pharmaceutique (1) selon l'une quelconque des revendications précédentes, dans laquelle le noyau (2) est constitué de

(A) 25 à 90 %, de préférence 40 à 90 % en poids dudit xyloglucane natif, hautement purifié, soluble dans l'eau froide ;
(B) 10 à 60 % en poids d'au moins un ingrédient pharmaceutique actif ;
(C) 0 à 20 %, de préférence 5 à 10 % en poids d'un ou plusieurs excipients pharmaceutiquement acceptables choisis dans le groupe constitué d'un diluant, d'un liant, d'un anti-adhérent, d'un lubrifiant, un agent glissant et une combinaison de ceux-ci, dans lequel de préférence l'excipient pharmaceutiquement acceptable consiste essentiellement en un liant ou un liant et un anti-adhérent, en particulier le liant étant choisi comme PVP et l'anti-adhérent comme stéarate de magnésium

ou dans lequel le noyau (2) est constitué de granules composés de

(A) 25 à 90 %, de préférence 40 à 90 % en poids dudit xyloglucane de type soluble dans l'eau froide, hautement purifié et natif ;
(B) 10 à 60 % en poids d'au moins un ingrédient pharmaceutique actif ;
(C) 0 à 20 %, de préférence 5 à 10 % en poids d'un ou plusieurs excipients pharmaceutiquement acceptables choisis dans le groupe constitué d'un diluant, d'un liant, d'un lubrifiant, d'un agent glissant et d'une combinaison de ceux-ci, dans lequel, de préférence, l'excipient pharmaceutiquement acceptable consiste essentiellement en un liant, en particulier le liant étant choisi parmi le PVP,

lesdits granulés étant compactés pour former un noyau (2) avant l'application de l'enveloppe, dans lequel, de préférence, avant le compactage, les granulés sont mélangés avec un anti-adhérent, de préférence sous la forme de stéarate de magnésium.

10. Forme posologique de formulation pharmaceutique (1) selon l'une quelconque des revendications précédentes, dans laquelle le rapport pondéral entre la matrice du noyau (2) et le ou les ingrédients pharmaceutiques actifs est d'au moins 1:2, de préférence d'au moins 1:1, et plus préférablement d'au moins 2:1.

11. Forme posologique de formulation pharmaceutique (1) selon l'une quelconque des revendications précédentes, adaptée à l'établissement, au rétablissement et/ou à la modification de l'équilibre de la population microbienne dans le côlon ou de la physiologie du tractus gastro-intestinal inférieur.

12. Forme posologique de formulation pharmaceutique (1) selon l'une quelconque des revendications précédentes, destinée à être utilisée comme immunomodulateur ou immunosuppresseur ou pour le traitement d'au moins l'une des affections suivantes : maladie inflammatoire de l'intestin, en particulier colite ulcéreuse et/ou maladie de Crohn, infection à Clostridium difficile, cancer du côlon, traitement postopératoire du côlon.

13. Forme posologique de formulation pharmaceutique (1) selon l'une quelconque des revendications précédentes, dans laquelle le principe actif pharmaceutique est choisi dans le groupe constitué par : la mésalazine, la budésonide, la capécitabine, le fluorouracile, l'irinotécan, l'oxaliplatine, 1' UFT, le cétuximab, le panitumumab, des ingrédients immunomodulateurs ou immunosuppresseurs, y compris des glucocorticoïdes immunosuppresseurs, des cytostatiques immunosuppresseurs, des anticorps immunosuppresseurs (poly- ou monoclonaux), des médicaments immunosuppresseurs agissant sur les immunophilines, les interleukines, les cytokines, chimiokines, médicaments imides immunomodulateurs, notamment le tacrolimus, la cyclosporine ou des substances appropriées pour établir, rétablir et/ou modifier l'équilibre de la population microbienne dans le côlon, ou des composés ayant un effet bénéfique sur la physiologie du tractus gastro-intestinal inférieur, ou des combinaisons de ceux-ci.

14. Forme posologique de formulation pharmaceutique (1) selon l'une quelconque des revendications précédentes, adaptée à une administration par voie orale, dans laquelle elle est administrée par voie orale au moins une fois par jour, de préférence deux fois par jour, pendant une durée d'au moins une semaine, de préférence d'au moins deux semaines, ou d'au moins deux mois ou d'au moins un an, voire à vie.

15. Procédé de fabrication d'une forme posologique de formulation pharmaceutique selon l'une quelconque des revendications précédentes, dans lequel, dans une première étape, ledit xyloglucane natif, hautement purifié, soluble dans l'eau froide, au moins un ingrédient pharmaceutique actif, ainsi que, si nécessaire, un ou plusieurs excipients pharmaceutiquement acceptables sont mélangés puis compactés pour former le noyau ou mélangés et traités pour former des granules, de préférence comprimés en un noyau unique ayant une densité relative d'au moins 0,7, de préférence d'au moins 0,75 ou d'au moins 0,8 et/ou ayant un diamètre moyen dans la direction du plus petit diamètre d'au moins 3 mm, de préférence d'au moins 4 mm, de préférence d'au moins 4,5 ou 5 mm, qui sont ensuite, si nécessaire, d'abord mélangés avec un autre agent de traitement, puis compactés pour former le noyau, le mélange pouvant dans les deux cas être effectué de préférence à l'aide d'un granulateur à lit fluidisé ou d'un mélangeur à haut cisaillement,

et le noyau est ensuite enrobé dans une deuxième étape avec au moins un enrobage formant une enveloppe, dans lequel de préférence la formulation d'enrobage est fournie sous forme de dispersion et est appliquée de préférence dans un enrobeur à tambour.

**FIG. 1**

FIG. 2

Accelerated drug release by microbial enzyme

FIG. 3

Drug release un-coated Xyloglucan tablets with different properties pH 7 (n=3)
200 mg 5-ASA total weight approx. 650 mg

— Breaking strength= 30 N Porosity= 28.7 %

— Breaking strength= 40 N Porosity= 25.3 %

— Breaking strength= 134 N Porosity= 14.8 %

FIG. 4

EP 3 846 784 B1

Drug release from coated tablets (in %) (n=3)

FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015158771 A
- US 2017088557 A
- WO 2007122374 A
- US 2018000740 A
- WO 2012038898 A
- WO 2009047802 A
- EP 3257501 A

### Non-patent literature cited in the description

- **PAULRAJ et al.** Bioinspired capsules based on nano-cellulose, xyloglucan and pectin - The influence of capsule wall composition on permeability properties. *Acta Biomaterialia*, 2018, vol. 69, 196-205
- **MISHRA et al.** *Int J Pharm Sci*, vol. 3 (1), 139-142
- **SVAGAN et al.** Rhamnogalacturonan-I Based Microcapsules for Targeted Drug Release. *PLOS ONE*, 19 December 2016
- **YOO et al.** *Arch Pharm Res*, 2005, vol. 28 (6), 736-742
- **BRUN-GRAEPPI ; AMANDA K. ANDRIOLA SILVA et al.** Study on the Sol-Gel Transition of Xyloglucan Hydrogels.. *Carbohydrate Polymers*, 2010, vol. 80 (2), 555-62
- **MAGDULKAR et al.** *Journal of Pharmaceutics*, 01 January 2013, vol. 2013, 1-11
- **PANDIT et al.** Drug Delivery Letters. Bentham Science Publishers B.V., 01 July 2015, vol. 5, 63-71
- **MISHRA ; MADHYA PRADESH, IN et al.** *International Journal of Pharmacy and Pharmaceutical Sciences*, 01 January 2011, vol. 3 (1), 139-142
- **NEWTON et al.** *International Journal of Biological Macromolecules*, 01 August 2015, vol. 79, 290-299